# EUROPEAN PATENT APPLICATION

(11) **EP 4 389 753 A1**
(43) Date of publication of application: **26.06.2024**
(21) Application number: 22858504.8
(22) Date of filing: 18.08.2022
(51) Int. Cl.: C07D 519/00, H05B 33/04, H10K 50/00, H05B 33/10

(54) **ORGANIC ELECTROLUMINESCENT ELEMENT AND COMPOUND FOR SAME**

(30) Priority: 20.08.2021 JP 2021134493
(71) Applicant: Hodogaya Chemical Co., Ltd., Tokyo, 105-0021 (JP)
(72) Inventor: CHIBA, Eriko, Tokyo 105-0021 (JP); KASE, Kouki, Tokyo 105-0021 (JP); YAMAMOTO, Takeshi, Tokyo 105-0021 (JP); HIRAYAMA, Yuta, Tokyo 105-0021 (JP); HAYASHI, Shuichi, Tokyo 105-0021 (JP)
(74) Representative: Müller-Boré & Partner Patentanwälte PartG mbB
(86) International application number: PCT/JP2022/031165
(87) International publication number: WO 2023/022186

(57) **Abstract**

An object of the present invention is to provide a compound not affecting a material inside a device by absorbing light at a wavelength of 400 nm to 410 nm of sunlight, and having a high refractive index at a wavelength in range of 450 nm to 750 nm in order to prevent deterioration inside the device of an organic EL device and greatly improve light extraction efficiency.

The present invention was achieved by focusing on the fact that arylamine-based materials have excellent thin film stability and durability and planning a diamine compound having a specific benzazole ring structure with a high refractive index, using the compound as materials constituting a capping layer, and an organic EL device having excellent luminous efficiency was obtained.

## Description

### Technical Field

The present invention relates to compounds suitable for a preferred self-luminous electron device for various display devices, and in particular, for an organic electroluminescent device (hereinafter referred to as organic EL device), and to an organic EL device, an electronic apparatus or an electron device using the compound.

### Background Art

The organic EL device is a self-luminous device and has been actively studied for their brighter, superior visibility and the ability to display clearer images in comparison with liquid crystal devices.

In 1987, C.W. Tang and colleagues at Eastman Kodak developed a laminated structure device using materials assigned with different roles, realizing practical applications of an organic EL device with organic materials. These researchers laminated an electron-transporting phosphor and a hole-transporting organic substance and injected both charges into a phosphor layer to cause emission in order to obtain a high luminance of 1,000 cd/m² or more at a voltage of 10 V or less (see, for example, PTLs 1 and 2).

To date, many improvements have been made for the practical application of organic EL devices. Various roles of the laminated structure have been further subdivided to provide a light emitting device that includes an anode, a hole injection layer, a hole transport layer, a light emitting layer, an electron transport layer, an electron injection layer, and a cathode sequentially on a substrate. In this light emitting device, efficiency and durability have come to be achieved by providing thereto a bottom emission structure that emits light from the bottom (see, for example, NPL 1).

Recently, a light emitting device with a top emission structure in which a metal having a high work function is used for an anode and light is emitted from the top is coming into use. In a light emitting device with a bottom-emission structure where light is taken out from the bottom having a pixel circuit, the area of the light emitting portion is limited, whereas in a light-emitting devices with a top-emission structure have the advantage that the light is taken out from the top and is not blocked by the pixel circuit, thus allowing a larger emission area. In the light emitting device with a top emission structure, translucent electrodes made of LiF/Al/Ag (see, for example, NPL 2), Ca/Mg (see, for example, NPL 3), LiF/MgAg, or the like are used for a cathode.

In such light emitting device, when light that is emitted by a light emitting layer and incident on another layer is incident at a certain angle or more, the light is totally reflected at an interface between the light emitting layer and the other layer. Thus, light that can be used has been limited to only a part of the emitted light. Recently, a light emitting device has been proposed in which a "capping layer" with a high refractive index is provided on the outside of a translucent electrode with a low refractive index, in order to improve the light extraction efficiency (see, for example, NPLs 2 and 3).

Regarding the effect of the capping layer in a light emitting device with a top emission structure, while a light emitting device using Ir(ppy)₃ as a light emitting material has a current efficiency of 38 cd/A in the case of not having a capping layer, the light emitting device has a current efficiency of 64 cd/A, in the case of using a ZnSe film with a thickness of 60 nm as a capping layer, which indicates that the efficiency is improved about 1.7 times. Furthermore, it is indicated that the maximum point of transmittance of the translucent electrode and capping layer does not necessarily coincide with the maximum point of efficiency, and that the maximum point of light extraction efficiency is determined by interference effects (see, for example, NPL 3).

In the past, it has been proposed to use a metal mask with a high degree of definition to form the capping layer, but there is a problem that the metal mask is distorted by heat when used under high temperature conditions, resulting in a decrease in alignment accuracy. Therefore, ZnSe has a high melting point of 1100°C or higher (see, for example, NPL 3), and a high definition metal mask cannot be used to deposit ZnSe at precise positions, which may affect the light emitting devices themselves. Furthermore, even deposition by the sputtering method affects the light emitting devices, inorganic materials are not suitable for use as components of the capping layer.

In addition, when tris(8-hydroxyquinoline) aluminum (Alq₃) is used as a capping layer to adjust the refractive index (see, for example, NPL 2), Alq₃ is known as an organic EL material commonly used as a green emitting material or electron transport material, it has a weak absorption around 450 nm, which is used for blue emitting materials, and has the problems of reducing the color purity and light extraction efficiency of blue emitting devices.

In addition, the devices fabricated with conventional capping layers allow light of 400 nm to 410 nm wavelength of sunlight to pass through, affecting the materials inside the devices and resulting in a decrease in color purity and light extraction efficiency.

In order to improve the device characteristics of organic EL device, especially to absorb light at a wavelength of 400 nm to 410 nm of sunlight, not to affect the materials inside the device, and to significantly improve the light extraction efficiency, materials with high absorption coefficient, high refractive index, and excellent thin film stability and durability are required as capping layer materials.

### Citation List

### Patent literatures

PTL 1: JP-A-Hei-8-048656
PTL 2: Japanese Patent No. 3194657
PTL 3: WO 2014/009310
PTL 4: WO 2013/038627

### Non-Patent Literatures

NPL 1: The Japan Society of Applied Physics, 9th Lecture Preprints, pp. 55-61 (2001)
NPL 2: Appl. Phys. Let., 78, 544 (2001)
NPL 3: Appl. Phys. Let., 82, 466 (2003)
NPL 4: SYNLETT., 7, 1172 (2009)
NPL 5: J. Org. Chem., 60, 7508 (1995)
NPL 6: Synth. Commun., 11, 513 (1981)
NPL 7: Appl. Phys. Lett., 98, 083302 (2011)

### Summary of the Invention

### Technical Problem

An object of the present invention is to improve the device characteristics of an organic EL device, in particular, to provide a compound not affecting the materials inside the device by absorbing light at a wavelength of 400 nm to 410 nm of sunlight, and having high refractive index at a wavelength in range of 450 nm to 750 nm, and it is to provide an organic EL device preventing deterioration inside the device and greatly improves light extraction efficiency by using the compound for a material of a capping layer of an organic EL device.

Specifically, physical properties of the material of the capping layer suitable for an organic EL device include (1) high absorption coefficient, (2) high refractive index, (3) vapor-deposit ability, (4) good thin film stability, and (5) high glass transition point. Properties of the organic EL device provided by the present invention include (1) absorption of light with a wavelength of 400 nm to 410 nm, (2) high light extraction efficiency, (3) no loss in color purity, (4) light transmission without changes over time, and (5) a long lifetime.

### Solution to Problem

For achieving the object, the present inventors have focused on the fact that arylamine-based materials have excellent thin film stability and durability, and from a diamine compound having a specific benzazole ring structure with a high refractive index, planned materials having high absorbance in the absorption spectrum with a concentration of 10⁻⁵ mol/L at a wavelength of 400 nm to 410 nm, and fabricated organic EL devices using the compounds as the materials constituting the capping layer, and thoroughly evaluated the properties of the devices, as a result of which the present invention has been accomplished.

Specifically, according to the present invention, the following diamine compounds having an azabenzooxazole ring structure and organic EL devices, electronic apparatuses or electron devices are provided.
1) A diamine compound having an azabenzooxazole ring structure represented by the following general formula (a-1) .

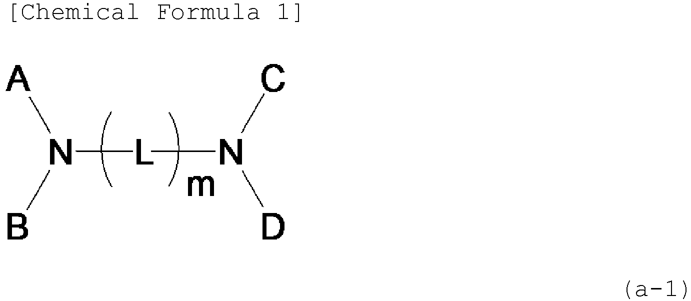

In the general formula (a-1), A, B, C and D may be the same or different, and represent a monovalent group having an azabenzooxazole group represented by the following structural formula (b-1), a substituted or unsubstituted aromatic hydrocarbon group, a substituted or unsubstituted aromatic heterocyclic group, or a substituted or unsubstituted condensed polycyclic aromatic group. At least one of A, B, C and D represents a monovalent group having an azabenzooxazole group represented by the following structural formula (b-1). L represents a divalent group of a substituted or unsubstituted aromatic hydrocarbon, a divalent group of a substituted or unsubstituted aromatic heterocyclic ring, or a divalent group of a substituted or unsubstituted condensed polycyclic aromatic, and may bind to each other with A, B, C or D via a single bond, a substituted or unsubstituted methylene, an oxygen atom, or a sulfur atom to form a ring. m represents an integer between 1 and 3, when m is an integer greater than or equal to 2, a plurality of L may be the same or different.

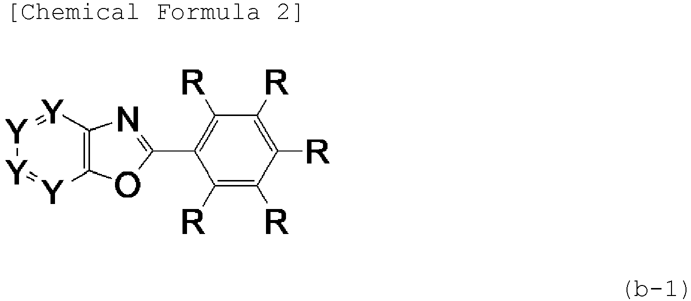

In the structural formula (b-1), Y may each be the same or different, and represents C-R or a nitrogen atom. At least one Y of a plurality of Y represents a nitrogen atom. R may each be the same or different, any one of a plurality of R is a linking group as a bonding site with the general formula (a-1), and represents a hydrogen atom, a deuterium atom, a chlorine atom, a cyano group, a nitro group, a trimethylsilyl group, a triphenylsilyl group, a linear or branched alkyl group of 1 to 6 carbon atoms that may have a substituent, a cycloalkyl group of 5 to 10 carbon atoms that may have a substituent, a linear or branched alkenyl group of 2 to 6 carbon atoms that may have a substituent, a linear or branched alkyloxy group of 1 to 6 carbon atoms that may have a substituent, a cycloalkyloxy group of 5 to 10 carbon atoms that may have a substituent, a substituted or unsubstituted aryloxy group, a substituted or unsubstituted aromatic hydrocarbon group, a substituted or unsubstituted aromatic heterocyclic group, or a substituted or unsubstituted condensed polycyclic aromatic group.

2) The diamine compound having an azabenzooxazole ring structure of 1), wherein A, B, C and D in the general formula (a-1) are a monovalent group having an azabenzooxazole group represented by the structural formula (b-1), a substituted or unsubstituted phenyl group, a substituted or unsubstituted biphenyl group, a substituted or unsubstituted naphthyl group, a substituted or unsubstituted phenanthrenyl group, a substituted or unsubstituted carbazolyl group, a substituted or unsubstituted benzofuranyl group, a substituted or unsubstituted benzothienyl group, a substituted or unsubstituted indolyl group, a substituted or unsubstituted benzoimidazolyl group, a substituted or unsubstituted imidazopyridyl group, a substituted or unsubstituted benzoxazolyl group, a substituted or unsubstituted benzothiazolyl group, a substituted or unsubstituted dibenzofuranyl group, or a substituted or unsubstituted dibenzothienyl group.

3) The diamine compound having an azabenzooxazole ring structure of 1) or 2), wherein the structural formula (b-1) is the following structural formula (b-2) or the following structural formula (b-3).

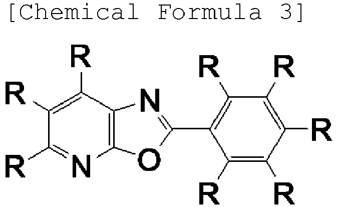

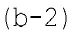

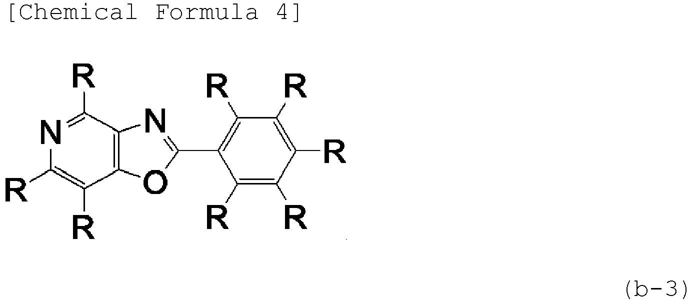

In the structural formula (b-2) or the structural formula (b-3), R is the same as defined by the structural formula (b-1).

4) The diamine compound having an azabenzooxazole ring structure of any one of 1) to 3), wherein L in the general formula (a-1) is a substituted or unsubstituted phenylene group, a substituted or unsubstituted biphenylene group, or a substituted or unsubstituted naphthylene group.

5) The diamine compound having an azabenzooxazole ring structure of any one of 1) to 4), wherein m in the general formula (a-1) is 1 or 2.

6) The diamine compound having an azabenzooxazole ring structure of any one of 1) to 5), wherein the structural formula (b-1) is the following structural formula (b-4) or the following structural formula (b-5).

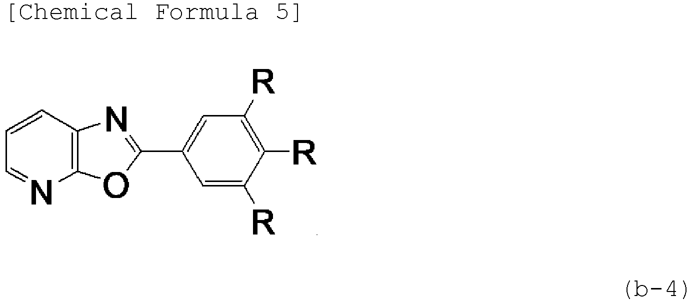

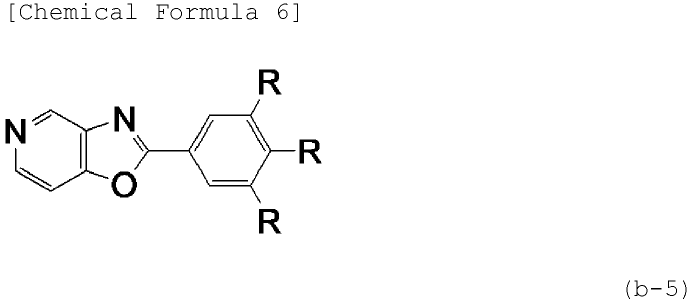

In the structural formula (b-4) or the structural formula (b-5), R is the same as defined by the structural formula (b-1).

7) The diamine compound having an azabenzooxazole ring structure of any one of 1) to 6), wherein only one of A, B, C and D in the general formula (a-1) is the structural formula (b-4) or the structural formula (b-5).

8) The diamine compound having an azabenzooxazole ring structure of any one of 1) to 6), wherein any two of A, B, C and D in the general formula (a-1) are the structural formula (b-4) or the structural formula (b-5).

9) The diamine compound having an azabenzooxazole ring structure of any one of 1) to 6), wherein A and B in the general formula (a-1) are the structural formula (b-4) or the structural formula (b-5).

10) The diamine compound having an azabenzooxazole ring structure of any one of 1) to 6), wherein A and C in the general formula (a-1) are the structural formula (b-4) or the structural formula (b-5).

11) An organic EL device comprising at least an anode electrode, a hole transport layer, a light emitting layer, an electron transport layer, a cathode electrode, and a capping layer in this order, wherein the capping layer includes the diamine compound having an azabenzooxazole ring structure of any one of 1) to 10).

12) The organic EL device of 11), wherein the capping layer has an extinction coefficient of 0.2 or higher at a wavelength in a range of 400 nm to 410 nm, and the diamine compound having an azabenzooxazole ring structure has an absorbance of 0.2 or higher in the absorption spectrum with a concentration of 10⁻⁵ mol/L at a wavelength in a range of 400 nm to 410 nm.

13) The organic EL device of 11) or 12), wherein the capping layer has a refractive index of 1.85 or higher while light transmitted through the capping layer having a wavelength in a range of 450 to 750 nm.

14) The organic EL device of any one of 11) to 13), wherein in the case where the capping layer is a laminate or a mixed layer comprising two or more compounds, at least one of the compounds is the diamine compound having an azabenzooxazole ring structure.

15) An electronic apparatus or an electron device comprising a pair of electrodes and at least one organic layer sandwiched therebetween, wherein the organic layer includes the diamine compound having an azabenzooxazole ring structure of any one of 1) to 10).

The "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by A, B, C, D, and R in the general formula (a-1) or the structural formula (b-1) may be specifically selected from a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, a triphenylenyl group, a pyridyl group, a pyrimidinyl group, a triazinyl group, a furyl group, a pyrrolyl group, a thienyl group, a quinolyl group, an isoquinolyl group, a benzofuranyl group, a benzothienyl group, an indolyl group, a carbazolyl group, an imidazopyridyl group, a benzoxazolyl group, a benzothiazolyl group, a quinoxalinyl group, a benzoimidazolyl group, a pyrazolyl group, a dibenzofuranyl group, a dibenzothienyl group, a naphthyridinyl group, a phenanthrolinyl group, an acridinyl group, and a carbolinyl group, and may also be selected from an aryl group of 6 to 30 carbon atoms and a heteroaryl group of 2 to 20 carbon atoms.

Specific examples of the "aromatic hydrocarbon", the "aromatic heterocyclic group", or the "condensed polycyclic aromatic" in the "divalent group of an aromatic hydrocarbon", the "divalent group of an aromatic heterocyclic group", or the "divalent group of condensed polycyclic aromatic" of the "divalent group of a substituted or unsubstituted aromatic hydrocarbon", the "divalent group of a substituted or unsubstituted aromatic heterocyclic group", or the "divalent group of a substituted or unsubstituted condensed polycyclic aromatic" represented by L in the general formula (a-1) include benzene, biphenyl, terphenyl, naphthalene, anthracene, phenanthrene, fluorene, pyridine, pyrimidine, quinoline, benzofuran, benzothiophene, benzoxazole, benzothiazole, dibenzofuran, dibenzothiophene, phenanthroline. Then, the "divalent group of an aromatic hydrocarbon", the "divalent group of an aromatic heterocyclic group", or the "divalent group of condensed polycyclic aromatic" in the "divalent group of a substituted or unsubstituted aromatic hydrocarbon", the "divalent group of a substituted or unsubstituted aromatic heterocyclic group", or the "divalent group of a substituted or unsubstituted condensed polycyclic aromatic" represented by L in the general formula (a-1) is a divalent group that results from the removal of two hydrogen atoms from the "aromatic hydrocarbon", the "aromatic heterocyclic ring", or the "condensed polycyclic aromatic" above.

Specific examples of the "linear or branched alkyl group of 1 to 6 carbon atoms", the "cycloalkyl group of 5 to 10 carbon atoms", the "linear or branched alkenyl group of 2 to 6 carbon atoms", the "linear or branched alkyloxy group of 1 to 6 carbon atoms", the "cycloalkyloxy group of 5 to 10 carbon atoms" or the "aryloxy group" in the "linear or branched alkyl group of 1 to 6 carbon atoms that may have a substituent", the "cycloalkyl group of 5 to 10 carbon atoms that may have a substituent", the "linear or branched alkenyl group of 2 to 6 carbon atoms that may have a substituent", the "linear or branched alkyloxy group of 1 to 6 carbon atoms that may have a substituent", the "cycloalkyloxy group of 5 to 10 carbon atoms that may have a substituent" or the "substituted or unsubstituted aryloxy group" represented by R in the structural formula (b-1) include a methyl group, an ethyl group, an n-propyl group, an isopropyl group, an n-butyl group, an isobutyl group, a tert-butyl group, an n-pentyl group, an isopentyl group, a neopentyl group, an n-hexyl group, a cyclopentyl group, a cyclohexyl group, a 1-adamantyl group, a 2-adamantyl group, a vinyl group, an allyl group, an isopropenyl group, a 2-butenyl group, a methyloxy group, an ethyloxy group, an n-propyloxy group, a cyclopentyloxy group, a cyclohexyloxy group, a 1-adamantyloxy group, a phenyloxy group, a tolyloxy group, and a biphenyloxy group.

Specific examples of the "substituent" in the "substituted aromatic hydrocarbon group", the "substituted aromatic heterocyclic group", the "substituted condensed polycyclic aromatic group", the "divalent group of an aromatic hydrocarbon having a substituent", the "divalent group of an aromatic heterocyclic group having a substituent", the "divalent group of condensed polycyclic aromatic having a substituent", the "linear or branched alkyl group of 1 to 6 carbon atoms having a substituent", the "cycloalkyl group of 5 to 10 carbon atoms having a substituent", the "linear or branched alkenyl group of 2 to 6 carbon atoms having a substituent", the "linear or branched alkyloxy group of 1 to 6 carbon atoms having a substituent", the "cycloalkyloxy group of 5 to 10 carbon atoms having a substituent", or the "substituted aryloxy group" represented by A, B, C, D, L, and R in the general formula (a-1), or the structural formula (b-1) include a deuterium atom; a cyano group; a nitro group; a halogen atom, such as a fluorine atom, a chlorine atom, a bromine atom, and an iodine atom; a silyl group, such as a trimethylsilyl group and a triphenylsilyl group; a linear or branched alkyl group of 1 to 6 carbon atoms, such as a methyl group, an ethyl group, and a propyl group; a linear or branched alkyloxy group of 1 to 6 carbon atoms, such as a methyloxy group, an ethyloxy group, and a propyloxy group; an alkenyl group, such as a vinyl group and an allyl group; an aryloxy group, such as a phenyloxy group and a tolyloxy group; an arylalkyloxy group, such as a benzyloxy group, and a phenethyloxy group; an aromatic hydrocarbon group, or a condensed polycyclic aromatic group, such as a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, and a triphenylenyl group; and a pyridyl group, a thienyl group, a furyl group, a pyrrolyl group, a quinolyl group, an isoquinolyl group, a benzofuranyl group, a benzothienyl group, an indolyl group, a carbazolyl group, an imidazopyridyl group, a benzoxazolyl group, a benzothiazolyl group, a quinoxalinyl group, a benzoimidazolyl group, a pyrazolyl group, a dibenzofuranyl group, a dibenzothienyl group, and a carbolinyl group; and also include an aryl group of 6 to 30 carbon atoms and a heteroaryl group of 2 to 20 carbon atoms. These substituents may be further substituted with the exemplified substituents above. The substituted aromatic rings with these substituents, or plural substituents on the same aromatic ring may bind to each other via a single bond, a substituted or unsubstituted methylene, an oxygen atom, or a sulfur atom to form a ring.

L in the amine compound having an azabenzooxazole ring structure represented by the general formula (a-1) of the present invention is preferably a divalent group that results from the removal of two hydrogen atoms from a substituted or unsubstituted benzene (i.e., a phenylene group), a divalent group that results from the removal of two hydrogen atoms from a substituted or unsubstituted biphenyl (i.e., a biphenylene group), or a divalent group that results from the removal of two hydrogen atoms from a substituted or unsubstituted naphthalene (i.e., a naphthylene group), more preferably, a divalent group that results from the removal of two hydrogen atoms from an unsubstituted benzene (i.e., a phenylene group), a divalent group that results from the removal of two hydrogen atoms from an unsubstituted biphenyl (i.e., a biphenylene group), or a divalent group that results from the removal of two hydrogen atoms from an unsubstituted naphthalene (i.e., a naphthylene group).

In the amine compound having an azabenzooxazole ring structure represented by the general formula (a-1) of the present invention, L may bind to each other with A, B, C or D via a single bond, a substituted or unsubstituted methylene, an oxygen atom, or a sulfur atom to form a ring.

In the amine compound having the azabenzooxazole ring structure represented by the general formula (a-1) of the present invention, m that is the number of L represents an integer from 1 to 3, it is preferred that m is 1 or 2. In the case where m is an integer more than 2, a plurality of L may be the same or different.

In the amine compound having an azabenzooxazole ring structure represented by the general formula (a-1) of the present invention, the "aromatic hydrocarbon group", the "aromatic heterocyclic group" or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by A, B, C and D are preferably a phenyl group, a biphenyl group, a naphthyl group, a phenanthrenyl group, carbazolyl group, a benzofuranyl group, a benzothienyl group, an indolyl group, a benzoimidazolyl group, an imidazopyridyl group, a benzoxazolyl group, a benzothiazolyl group, a dibenzofuranyl group, or a dibenzothienyl group.

In the amine compound having an azabenzooxazole ring structure represented by the general formula (a-1) of the present invention, Y in the structural formula (b-1) may be the same or different, and is C-R or a nitrogen atom. At least one of the plurality of Y is a nitrogen atom.

In the amine compound having an azabenzooxazole ring structure represented by the general formula (a-1) of the present invention, at least one of A, B, C and D is the structural formula (b-1), and only one of A, B, C and D, or any two of A, B, C and D (for example, A and B, or A and C) are preferably the structural formula (b-1).

In the amine compound having an azabenzooxazole ring structure represented by the general formula (a-1) of the present invention, the structural formula (b-1) is preferably the structural formula (b-2), (b-3), (b-4), or (b-5), more preferably (b-4) or (b-5).

In the organic EL device of the present invention, the thickness of the capping layer is preferably in a range of 30 nm to 120 nm, and more preferably in a range of 40 nm to 80 nm.

In the organic EL device of the present invention, the refractive index of the capping layer to light transmitted through the capping layer having a wavelength in a range of 450 nm and 750 nm is preferably 1.85 or higher, more preferably 1.88 or higher, and further preferably 1.90 or higher.

In the organic EL device of the present invention, the extinction coefficient of the capping layer to light having a wavelength in a range of 400 nm and 410 nm is preferably 0.20 or higher, and more preferably 0.40 or higher, further preferably 0.80 or higher.

In the organic EL devices of the present invention, the diamine compound having an azabenzooxazole ring structure represented by the general formula (a-1) preferably used has an absorbance of 0.2 or higher in the absorption spectrum with a concentration of 10⁻⁵ mol/L at a wavelength in a range of 400 nm to 410 nm, more preferably 0.3 or higher, further preferably 0.4 or higher.

In the organic EL devices of the present invention, the capping layer may be fabricated by a laminate or a mixed layer comprising two or more different components. In this case, at least one of the components is preferably the diamine compound having an azabenzooxazole ring structure represented by the general formula (a-1) of the present invention.

### Advantageous Effects of Invention

The diamine compound having an azabenzooxazole ring structure represented by the general formula (a-1) of the present invention has (1) high absorption coefficient, (2) high refractive index at a wavelength in a range of 450 nm to 750 nm, (3) vapor-deposit ability, (4) stability in thin film state, (5) high heat resistance. Therefore, the compound is used as a capping layer with a higher refractive index than that of an electrode in the case where the capping layer is provided on the outside of a transparent or a translucent electrode of an organic EL device, making it possible to obtain an organic EL device that can significantly improve the light extraction efficiency, prevent material deterioration inside the device, and improve luminance, luminous efficiency, power efficiency and lifetime.

### Brief Description of Drawings

FIG. 1 is a figure showing the structure of the compounds (1-1) to (1-12) as the diamine compound having an azabenzooxazole ring structure represented by the general formula (a-1) of the present invention.
FIG. 2 is a figure showing the structure of the compounds (1-13) to (1-23) as the diamine compound having an azabenzooxazole ring structure represented by the general formula (a-1) of the present invention.
FIG. 3 is a figure showing the structure of the compounds (1-24) to (1-35) as the diamine compound having an azabenzooxazole ring structure represented by the general formula (a-1) of the present invention.
FIG. 4 is a figure showing the structure of the compounds (1-36) to (1-47) as the diamine compound having an azabenzooxazole ring structure represented by the general formula (a-1) of the present invention.
FIG. 5 is a figure showing the structure of the compounds (1-48) to (1-57) as the diamine compound having an azabenzooxazole ring structure represented by the general formula (a-1) of the present invention.
FIG. 6 is a figure showing the structure of the compounds (1-58) to (1-69) as the diamine compound having an azabenzooxazole ring structure represented by the general formula (a-1) of the present invention.
FIG. 7 is a diagram illustrating the configuration of the organic EL devices of Examples 10 to 15 and Comparative Examples 1 to 2.

### Description of Embodiments

The diamine compounds having an azabenzooxazole ring structure represented by the general formula (a-1) of the present invention are novel compounds, the azabenzooxazole derivative as the major skeleton of the compounds can be synthesized by the known methods as described below (see, for example, NPL 4). Furthermore, the diamine compound having an azabenzooxazole ring structure represented by the general formula (a-1) of the present invention can be synthesized by conducting coupling reaction between the synthesized halogenated azabenzooxazole derivative and an arylamine using a copper catalyst or a palladium catalyst. The diamine compound having an azabenzooxazole ring structure represented by the general formula (a-1) of the present invention can also be synthesized by derivatizing the halogenated azabenzooxazole derivative into a boronic acid ester derivative, followed by coupling reaction with a halogenated arylamine (see, for example, NPL 5 and NPL 6).

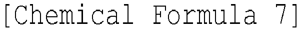

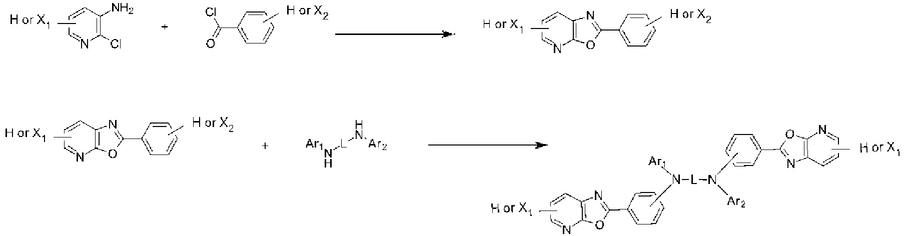

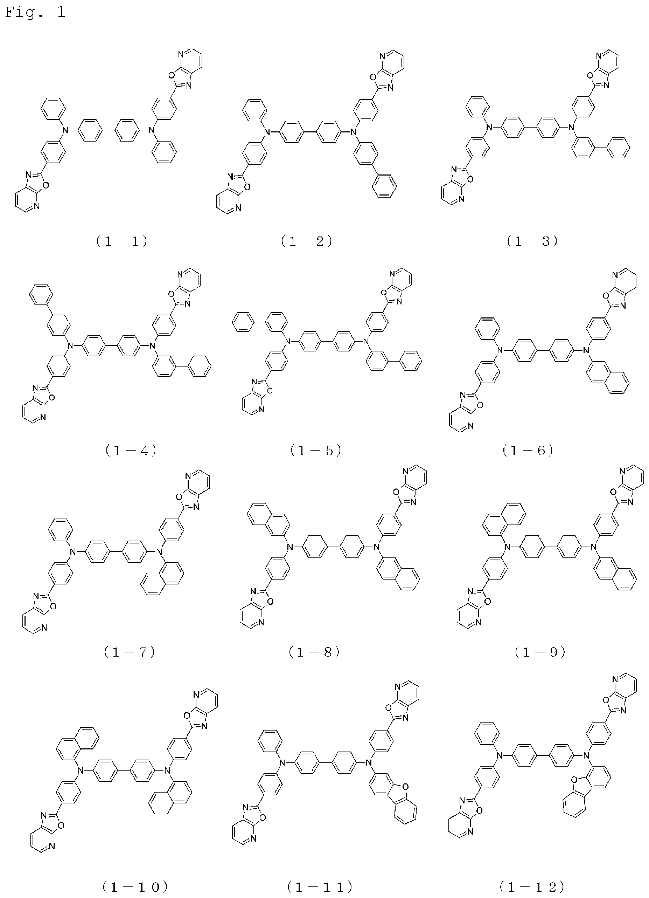

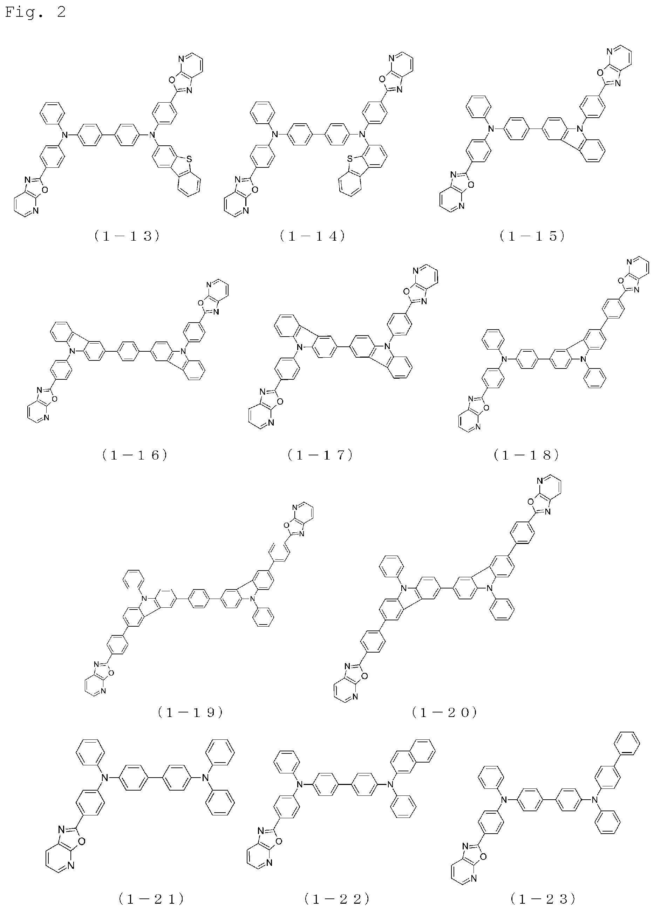

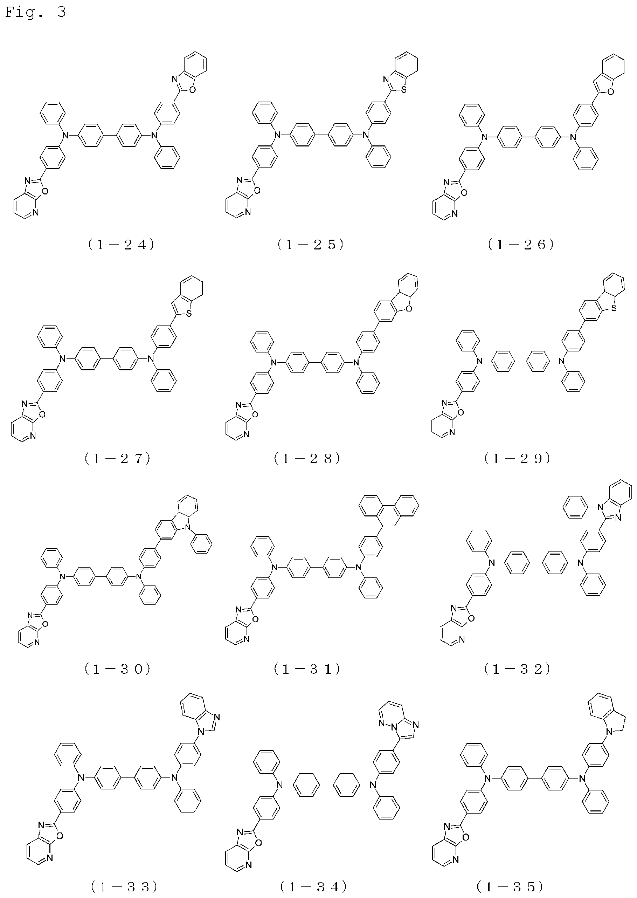

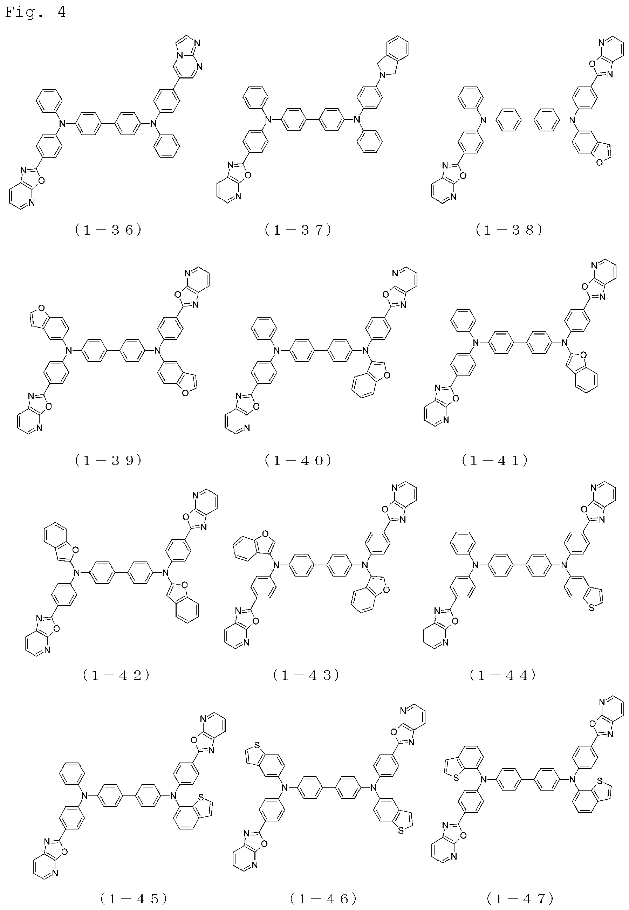

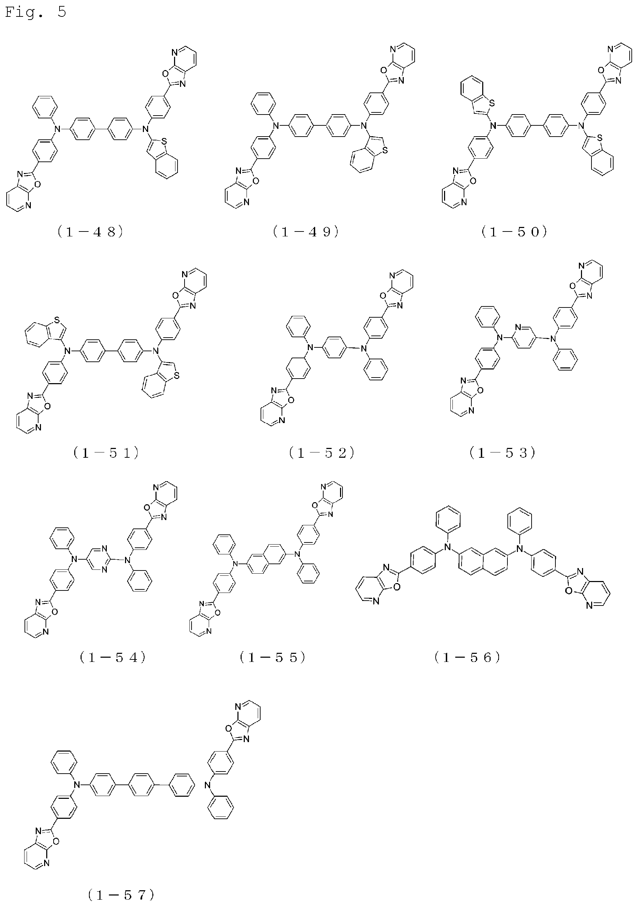

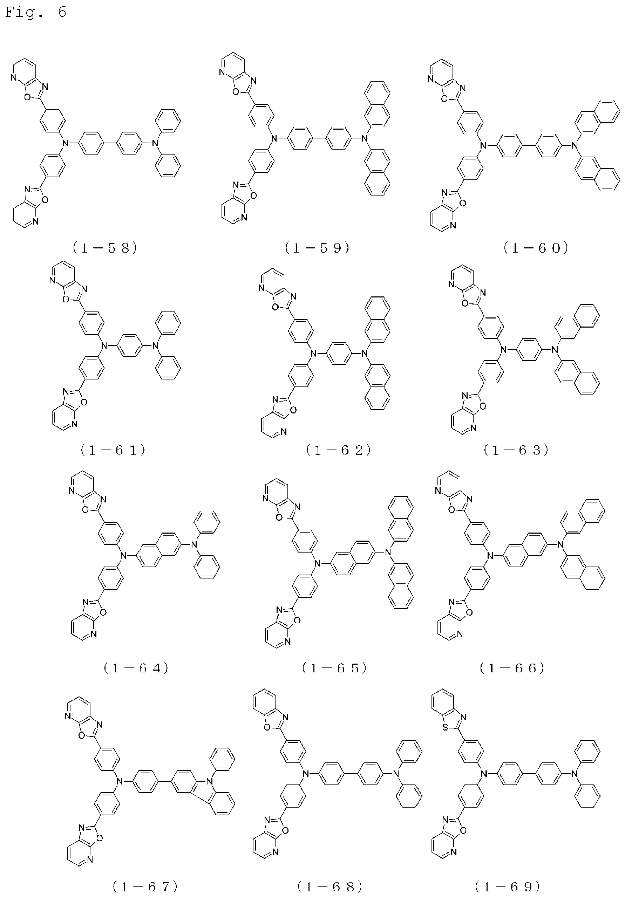

The specific examples of preferred compounds of the diamine compound having an azabenzooxazole ring structure represented by the general formula (a-1) of the present invention are shown in Figs 1 to 6. The present invention, however, is not restricted to these compounds.

The purification method of the diamine compound having an azabenzooxazole ring structure represented by the general formula (a-1) of the present invention is not particularly limited, the diamine compound is purified by known methods used for purification of organic compounds such as column chromatography, adsorption purification using, for example, a silica gel, activated carbon, or activated white clay, recrystallization purification method or crystallization purification method using a solvent, and finally, the diamine compound is purified by the sublimation purification method. The compound is identified by NMR analysis. The melting point and the glass transition point (Tg), the refractive index and the extinction coefficient, and the absorbance are measured as material property values. The melting point can be used as an index of the deposition properties, the glass transition point (Tg) can be used as an index of the stability in a thin film state, and the refractive index and the extinction coefficient can be used as an index of the enhancement of the light extraction efficiency, the absorbance can be used as an index of the color purity the light resistance.

The melting point and the glass transition point (Tg) are measured by using a powder specimen with a high sensitivity differential scanning calorimeter (DSC 3100SA, produced by Bruker AXS).

The refractive index and the extinction coefficient are measured in such a manner that a thin film of 80 nm is formed on a silicon substrate and measured with a spectrometer (F10-RT-UV, produced by Filmetrics).

The absorbance is measured in a toluene solvent at a prepared concentration of 10⁻⁵ mol/L, and the absorption coefficient is measured in a toluene solution at prepared four concentrations of 5.0 × 10⁻⁶ mol/L, 1.0 × 10⁻⁵ mol/L, 1.5 × 10⁻⁵ mol/L, and 2.0 × 10⁻⁵ mol/L with a UV-visible-near-infrared spectrophotometer (V-650, produced by JASCO Corporation).

The organic EL device of the present invention, for example, in the case of a top emission light emitting device, may have a structure including an anode, a hole transport layer, a light emitting layer, an electron transport layer, a cathode, and a capping layer successively formed on a glass substrate, optionally with a hole injection layer between the anode and the hole transport layer, an electron blocking layer between the hole transport layer and the light emitting layer, a hole blocking layer between the light emitting layer and the electron transport layer, and an electron injection layer between the electron transport layer and the cathode. Some of the organic layers in the multilayer structure may be omitted or may serve more than one function. For example, a single organic layer may serve as a hole injection layer and a hole transport layer, a hole transport layer and an electron blocking layer, a hole blocking layer and an electron transport layer, or an electron transport layer and an electron injection layer, and so on. Further, any of the layers may be configured to laminate two or more organic layers having the same function, and the hole transport layer may have a two-layer laminated structure, the light emitting layer may have a two-layer laminated structure, the electron transport layer may have a two-layer laminated structure, the capping layer may have a two-layer laminated structure, and so on.

The total thickness of each layer of the organic EL device is preferably 200 nm to 750 nm, more preferably 350 nm to 600 nm. The thickness of the capping layer is, for example, preferably 30 nm to 120 nm, and more preferably 40 nm to 80 nm. In this case, good light extraction efficiency can be obtained. The thickness of the capping layer can be changed according to the type of light-emitting material used in the light-emitting device, the thicknesses of the layers of the organic EL device other than the capping layer, and other factors.

A transparent electrode material with a large work function, such as ITO and gold, may be used as the anode of the organic EL device of the present invention.

The hole injection layer of the organic EL device of the present invention is preferably an arylamine compound having a structure in which two or more triphenylamine structures are joined within a molecule via a single bond or a divalent group that does not contain a heteroatom, for example, an arylamine compound having a structure in which two triphenylamine structures are joined within a molecule via a single bond or a divalent group that does not contain a heteroatom, such as a benzidine derivative, a starburst-type triphenylamine derivative, and various triphenylamine tetramers. A porphyrin compound, such as copper phthalocyanine; an accepting heterocyclic compound, such as hexacyanoazatriphenylene; and a coating-type polymer material may also be used. These materials may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin film by using a vapor deposition method or other known methods, such as a spin coating method and an inkjet method.

The hole transport layer of the organic EL device of the present invention is preferably a benzidine derivative, such as N,N'-diphenyl-N,N'-di(m-tolyl)benzidine (TPD), N,N'-diphenyl-N,N'-di(α-naphthyl)benzidine (NPD), and N,N,N',N'-tetrabiphenylylbenzidine; an arylamine compound having a structure in which two triphenylamine structures are joined within a molecule via a single bond or a divalent group that does not contain a heteroatom, such as 1,1-bis[4-(di-4-tolylamino)phenyl]cyclohexane (TAPC); and an arylamine compound having only one triphenylamine structure within a molecule. It is also preferable to use an arylamine compound having a structure in which three or more triphenylamine structures are joined within a molecule via a single bond or a divalent group that does not contain a heteroatom, such as various triphenylamine trimers or tetramers and the like. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. In addition, a coating-type polymer material, such as poly(3,4-ethylenedioxythiophene) (PEDOT)/poly(styrene sulfonate) (PSS) may be used as the injection and transport layer of the hole. These materials may be formed into a thin film by a vapor deposition method or other known methods such as a spin coating method and an inkjet method.

The material used for the hole injection layer or the hole transport layer is preferably obtained by p-doping a material commonly used for these layers with trisbromophenylaminehexachloroantimony, a radialene derivative (see, for example, PTL 3), or the like. A polymer compound having, as a part of the compound structure, a structure of a benzidine derivative, such as TPD, may also be used.

The electron blocking layer of the organic EL device of the present invention may be a compound having an electron blocking effect, including, for example, a carbazole derivative, such as 4,4',4''-tri(N-carbazolyl)triphenylamine (TCTA), 9,9-bis[4-(carbazol-9-yl)phenyl]fluorene, 1,3-bis(carbazol-9-yl)benzene (mCP), and 2,2-bis(4-carbazol-9-yl-phenyl)adamantane (Ad-Cz); and a compound having a triphenylsilyl group and a triarylamine structure, as represented by 9-[4-(carbazol-9-yl)phenyl]-9-[4-(triphenylsilyl)phenyl]-9H-fluorene. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin film by a vapor deposition method or other known methods such as a spin coating method and an inkjet method.

The light emitting layer of the organic EL device of the present invention may be metal complexes of a quinolinol derivative, such as Alq₃, as well as various metal complexes, an anthracene derivative, a bis-styrylbenzene derivative, a pyrene derivative, an oxazole derivative, a polyparaphenylene vinylene derivative, etc. Further, the light emitting layer may be made of a host material and a dopant material. An anthracene derivative is preferably used as the host material, but in addition to the light emitting materials above, a heterocyclic compound having an indole ring as a partial structure of the fused ring, a heterocyclic compound having a carbazole ring as a partial structure of fused ring, a carbazole derivative, a thiazole derivative, a benzimidazole derivative, a polydialkylfluorene derivative may be used. Further, as the dopant material, quinacridone, coumarin, rubrene, perylene and their derivatives, a benzopyran derivative, a rhodamine derivative, and an amino styryl derivative may be used. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer.

Further, the light-emitting material may be a phosphorescent material. Phosphorescent materials as metal complexes of metals such as iridium and platinum may be used. Examples of the phosphorescent materials include green phosphorescent materials such as Ir(ppy)₃, blue phosphorescent materials such as FIrpic and FIr6, and red phosphorescent materials such as Btp₂Ir(acac). Here, as the hole injecting and transporting host material, 4,4'-di(N-carbazolyl)biphenyl (CBP), and a carbazole derivative such as TCTA and mCP may be used. As electron transporting host materials, p-bis(triphenylsilyl)benzene (UGH2), 2, 2',2'-(1,3,5-phenylene)-tris(1-phenyl-1H-benzimidazole) (TPBI) may be used. In this way, a high-performance organic EL device can be produced.

To avoid concentration quenching, the doping of the host material with the phosphorescent light-emitting material should preferably be made by co-evaporation in a range of 1 to 30 weight percent with respect to the whole light emitting layer.

Further, Examples of the light-emitting material may be delayed fluorescent-emitting material such as a CDCB derivative of PIC-TRZ, CC2TA, PXZ-TRZ, 4CzIPN, or the like (see, for example, NPL 7). These materials may be formed into a thin film by using a vapor deposition method or other known methods such as a spin coating method and an inkjet method.

The hole blocking layer of the organic EL device of the present invention may be formed by using a hole blocking compound, such as phenanthroline derivatives such as bathocuproin (BCP), and the metal complexes of quinolinol derivatives such as aluminum(III) bis(2-methyl-8-quinolinate)-4-phenylphenolate (BAlq), various rare earth complexes, triazole derivatives, triazine derivatives, pyrimidine derivatives, oxadiazole derivatives, and benzazole derivatives. These materials may also serve as the material of the electron transport layer. These materials may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin film by using a vapor deposition method or other known methods, such as a spin coating method and an inkjet method.

The electron transport layer of the organic EL device of the present invention may be formed by using metal complexes of a quinolinol derivative, such as Alq₃, BAlq as well as various metal complexes, triazine derivatives, pyrimidine derivatives, oxadiazole derivatives, pyridine derivatives, benzimidazole derivatives, benzazole derivatives, thiadiazole derivatives, anthracene derivatives, carbodiimide derivatives, quinoxaline derivatives, pyridoindole derivatives, phenanthroline derivatives, and silole derivatives. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin film by using a vapor deposition method or other known methods such as a spin coating method and an inkjet method.

Examples of material used for the electron injection layer of the organic EL device of the present invention can be alkali metal salts such as lithium fluoride and cesium fluoride; alkaline earth metal salts such as magnesium fluoride; metal complexes of quinolinol derivatives such as lithium quinolinol; metal oxides such as aluminum oxide; and metals such as ytterbium (Yb), samarium (Sm), calcium (Ca), strontium (Sr), cesium (Cs). However, the electron injection layer may be omitted in the preferred selection of the electron transport layer and the cathode.

Further, in the electron injection layer or the electron transport layer, a material obtained by further N-doping a material that is commonly used for the layer with a metal such as cesium, or the like can be used.

The cathode of the organic EL device of the present invention may be made of an electrode material with a low work function such as aluminum, an alloy with an even lower work function such as a magnesium-silver alloy, a magnesium-calcium alloy, a magnesium-indium alloy, or an aluminum-magnesium alloy, or ITO or IZO. In the organic EL device having a top emission structure as an example of the present invention, the cathode in the direction from which light is extracted externally from the light emitting device is preferably transparent or translucent.

The amine compound having an azabenzoazole ring structure represented by the above general formula (a-1) is preferably used as the capping layer of the organic EL device of the present invention. It is preferable that the capping layer having the amine compound having an azabenzoazole ring structure represented by the above general formula (a-1) as constitutional material has the refractive index at a wavelength of from 450 nm to 750 nm of light transmitted through the capping layer of 1.85 or higher, and more preferably 1.88 or higher, further preferably 1.90 or higher.

In the case of using the amine compound having an azabenzoazole ring structure represented by the above general formula (a-1) of the present invention as the capping layer of the organic EL device, the compound may be individually deposited for film forming, may be used as a single layer deposited mixed with two or more compounds, or may be formed as a laminate of individually deposited layers, a laminate of mixedly deposited layers, or a laminate of the individually deposited layer and the mixedly deposited layer.

In the case where the capping layer of the organic EL device of the present invention is a laminate or a mixed layer comprising two or more compounds, at least one of the compounds as constitutional materials is preferably the diamine compound having an azabenzooxazole ring structure represented by the general formula (a-1) in the organic EL device. These materials may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin film by using a vapor deposition method or other known methods such as a spin coating method and an inkjet method.

The refractive index of the capping layer is preferably greater than the refractive index of the adjacent electrode. In other words, the capping layer improves the light extraction efficiency in the organic EL device, and the effect is more effective when the reflectance at the interface between the capping layer and the material in contact with the capping layer is larger because the effect of light interference is greater. Therefore, the refractive index of the capping layer is preferably greater than the refractive index of the adjacent electrode. The refractive index may be 1.70 or higher, preferably 1.85 or higher, more preferably 1.88 or higher, and further preferably 1.90 and more.

The above description refers to an organic EL device with a top emission structure, but the present invention is not limited to this, and can also be applied to organic EL devices with bottom emission structures, and organic EL devices with dual emission structures that emit light from both the top and the bottom. In these cases, the electrode in the direction from which light is extracted from the light emitting device to the outside is preferably transparent or translucent.

### Example

The following describes an embodiment of the present invention in more detail based on Examples. The present invention, however, is not restricted to the following Examples as long as they do not go beyond the gist of the invention.

### Example 1

### <Synthesis of Compound (1-1)>

Diphenylbenzidine: 6.2 g, 2-(4-bromophenyl)oxazolo[5,4-b]pyridine: 11.15 g, sodium-tert-butoxide: 3.9 g, palladium acetate (II) : 0.17 g, and tri-tert-butylphosphine(50%toluene solution): 0.3 g were added to a reaction vessel, and the mixture in toluene solution was stirred to reflux overnight. After the reaction was completed, dispersion washing was carried out to remove the insoluble matter by filtration, then the resulting filtrate was concentrated to obtain a crude product. The crude product was purified by crystallization using a monochlorobenzene/acetone mixed solvent, and yellow powder of a compound (1-1): 5.5g (yield: 41.2%) was obtained.

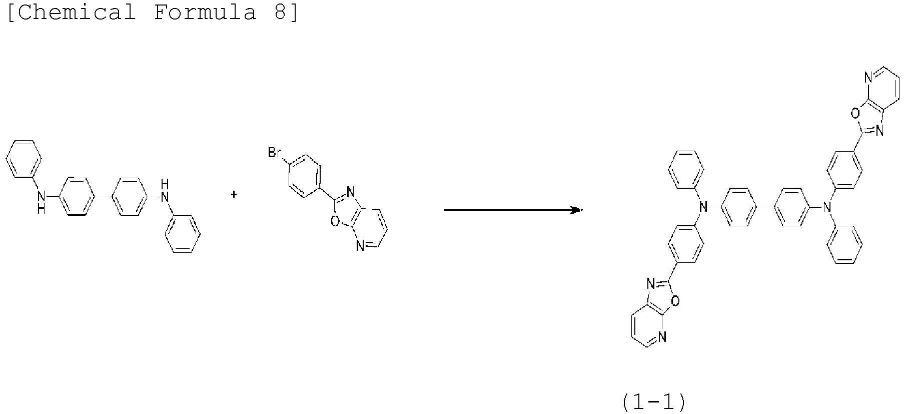

The structure of the obtained yellow powder was identified by NMR.

The ¹H-NMR (CDCl₃) detected 32 hydrogen signals, as follows.
δ (ppm) = 8.30-8.28 (2H), 8.13-8.11 (4H), 8.01-7.99 (2H), 7.56-7.54 (4H), 7.38-7.30 (6H), 7.26-7.22 (8H), 7.19-7.15 (6H).

### Example 2

### <Synthesis of Compound (1-24)>

phenyl-(4'-bromobiphenyl-4-yl)-4-(2-benzoxazolyl)phenylamine: 8.0g, phenyl-4-(oxazolo[5,4-b]pyridine-2-yl)phenylamine: 4.9g, sodium-tert-butoxide: 2.2g, tridibenzylideneacetonepalladium (0): 0.4g, and tri-tert-butylphosphine (50%toluene solution): 0.4g were added to a reaction vessel, and the mixture in toluene solution was stirred to reflux overnight. After the reaction was completed, the insoluble matter was removed by filtration, then the resulting filtrate was concentrated to obtain a crude product. The crude product was purified by column chromatography (a dichloromethane/ethyl acetate mixed solvent), and yellow powder of a compound (1-24): 5.6g (yield: 49.6%) was obtained.

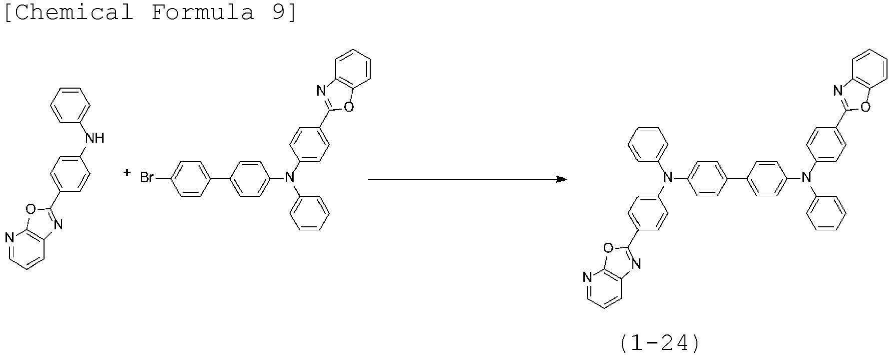

The structure of the obtained yellow powder was identified by NMR.

The ¹H-NMR (CDCl₃) detected 33 hydrogen signals, as follows.
δ(ppm) = 8.29 (1H), 8.11 (4H), 8.00 (1H), 7.73 (1H), 7.57-7.54 (5H), 7.38-7.30 (7H), 7.26-7.21 (8H), 7.19-7.16 (6H).

### Example 3

### <Synthesis of Compound (1-52)>

1-bromo-4-iodobenzene: 7.0g, phenyl-(4-oxazolo[5,4-b]pyridine-2-yl)phenylamine: 15.6g, sodium-tert-butoxide: 7.1g, tridibenzylideneacetonepalladium (0): 0.9g, and tri-tert-butylphosphine: 0.4g were added to a reaction vessel, and the mixture in toluene solution was stirred to reflux overnight. After the reaction was completed, dispersion washing was carried out to obtain a crude product. The crude product was purified by crystallization using a monochlorobenzene/acetone mixed solvent, and yellow powder of a compound (1-52): 8.8g (yield: 55.1%) was obtained.

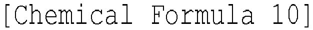

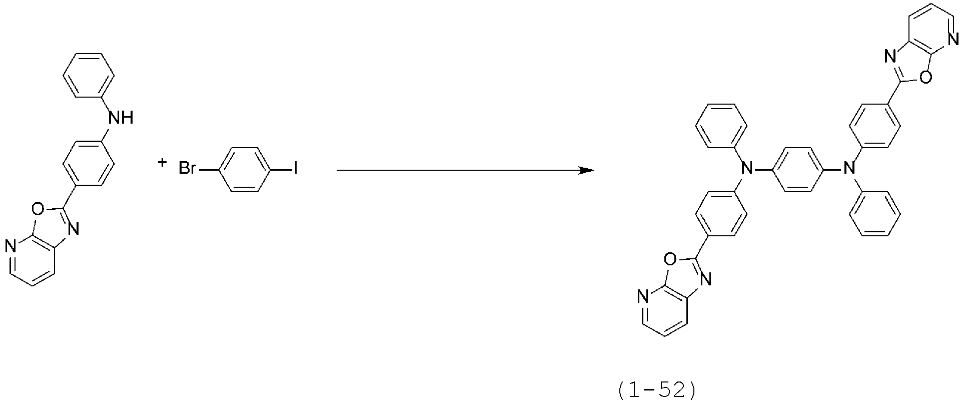

The structure of the obtained yellow powder was identified by NMR.

The ¹H-NMR (CDCl₃) detected 28 hydrogen signals, as follows.
δ(ppm) = 8.29 (2H), 8.12 (4H), 8.00 (2H), 7.38 (4H), 7.32 (2H), 7.23 (4H), 7.20-7.13 (10H).

### Example 4

### <Synthesis of Compound (1-55)>

2,6-dibromonaphthalene: 5.0g, phenyl-(4-oxazolo[5,4-b]pyridine-2-yl)phenylamine: 11.1g, sodium-tert-butoxide: 5.1g, tridibenzylideneacetonepalladium (0): 0.6g, and tri-tert-butylphosphine: 0.3g were added to a reaction vessel, and the mixture in toluene solution was stirred to reflux overnight. After the reaction was completed, dispersion washing was carried out to obtain a crude product. The crude product was purified by crystallization using a monochlorobenzene/acetone mixed solvent, and yellow powder of a compound (1-55): 7.9g (yield: 53.1%) was obtained.

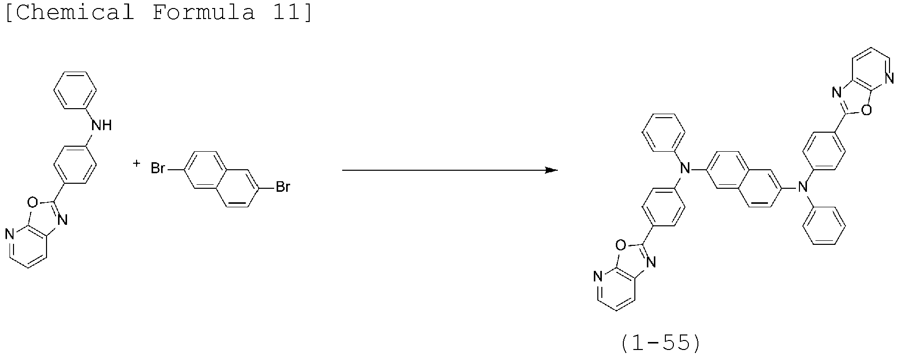

The structure of the obtained yellow powder was identified by NMR.

The ¹H-NMR (CDCl₃) detected 30 hydrogen signals, as follows.
δ(ppm) = 8.30 (2H), 8.12 (4H), 8.01 (2H), 7.64 (2H), 7.54 (2H), 7.38-7.29 (8H), 8.24 (4H), 7.19(6H).

### Example 5

### <Synthesis of Compound (1-56)>

2,7-dibromonaphthalene: 6.0g, phenyl-(4-oxazolo[5,4-b]pyridine-2-yl)phenylamine: 13.3g, sodium-tert-butoxide: 6.1g, tridibenzylideneacetonepalladium (0): 0.8g, and tri-tert-butylphosphine:0.3g were added to a reaction vessel, and the mixture in toluene solution was stirred to reflux overnight. After the reaction was completed, dispersion washing was carried out to obtain a crude product. The crude product was purified by crystallization using a monochlorobenzene/acetone mixed solvent, and yellow powder of a compound (1-56): 8.1g (yield: 55.1%) was obtained.

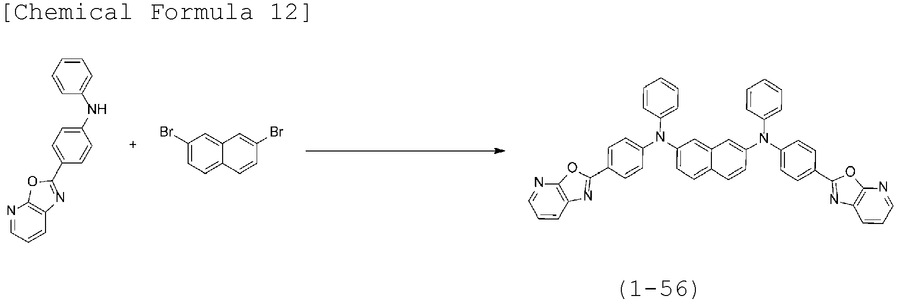

The structure of the obtained yellow powder was identified by NMR.

The ¹H-NMR (CDCl₃) detected 30 hydrogen signals, as follows.
δ(ppm) = 8.29 (2H), 8.10 (4H), 7.99 (2H), 7.75 (2H), 7.36-7.26 (10H), 7.21 (4H), 7.16(6H).

### Example 6

### <Synthesis of Compound (1-57)>

4,4"-diiodo-p-terphenyl: 7.1g, phenyl-(4-oxazolo[5,4-b]pyridine-2-yl)phenylamine: 9.2g, sodium-tert-butoxide: 4.2g, tridibenzylideneacetonepalladium (0): 0.4g, and tri-tert-butylphosphine(50%toluene solution): 0.3g were added to a reaction vessel, and the mixture in toluene solution was stirred to reflux overnight. After the reaction was completed, dispersion washing was carried out to remove the insoluble matter by filtration, then the resulting filtrate was concentrated to obtain a crude product. The crude product was purified by column chromatography (a dichloromethane/ethyl acetate mixed solvent), and yellow powder of a compound (1-57): 6.8g (yield: 58.6%) was obtained.

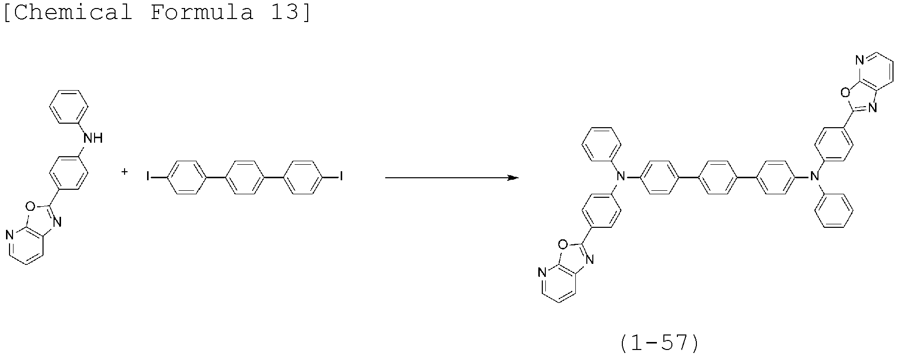

The structure of the obtained yellow powder was identified by NMR.

The ¹H-NMR (CDCl₃) detected 36 hydrogen signals, as follows.
δ(ppm) = 8.30 (2H), 8.13 (4H), 8.00 (2H), 7.68 (4H), 7.61 (4H), 7.37 (4H), 7.32 (2H), 7.27-7.22 (8H), 7.20-7.17 (6H).

### Example 7

The melting points and the glass transition points (Tg) of the diamine compound having an azabenzooxazole ring structure represented by the general formula (a-1) were determined with a high-sensitivity differential scanning calorimeter (DSC3100SA, produced by Bruker AXS). The measurement results are summarized below.

| | Melting point | Glass transition point (Tg) |
|---|---|---|
| Compound (1-1) | 279.6°C | 136.7°C |
| Compound (1-24) | not observed | 131.5°C |
| Compound (1-52) | 280.6°C | 120.4°C |
| Compound (1-55) | 301.0°C | 143.1°C |
| Compound (1-56) | 251.1°C | 141.6°C |
| Compound (1-57) | 300.2°C | 144.3°C |

The results show that the diamine compounds having an azabenzooxazole ring structure represented by the general formula (a-1) have glass transition points (Tg) of 100°C or higher, demonstrating that the compounds have a stable thin-film state.

### Example 8

An 80 nm-thick vapor-deposited film was fabricated on a silicon substrate using the diamine compounds having an azabenzooxazole ring structure represented by the general formula (a-1), and the refractive index n at wavelengths of 450 nm and 750 nm and the extinction coefficient k at wavelengths of 400 nm and 410 nm were measured with a spectrometer (F10-RT-UV, produced by Filmetrics). For comparison, the comparative compound (2-1) of the following structural formula and Alq₃ were also measured (see, for example, PTL 4). The measurement results are summarized in Table 1.

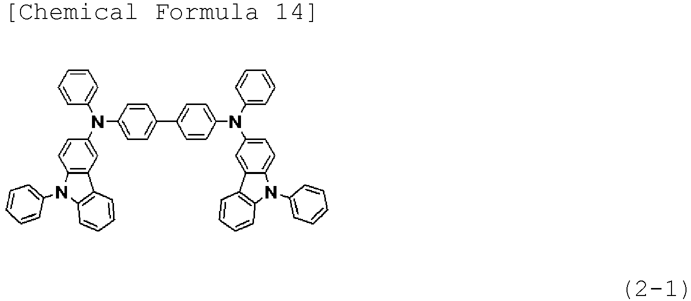

**[Table 1]**

| | Refractive index n | Refractive index n | Extinction coefficient k | Extinction coefficient k |
|---|---|---|---|---|
| | (λ:450 nm) | (λ:750 nm) | (λ:400 nm) | (λ:410 nm) |
| Compound (1-1) | 2.38 | 1.89 | 0.98 | 0.83 |
| Compound (1-24) | 2.35 | 1.88 | 1.01 | 0.80 |
| Compound (1-52) | 2.41 | 1.89 | 1.00 | 0.86 |
| Compound (1-55) | 2.58 | 1.92 | 1.08 | 1.05 |
| Compound (1-56) | 2.39 | 1.90 | 1.05 | 0.82 |
| Compound (1-57) | 2.42 | 1.89 | 1.09 | 0.93 |
| Alq₃ | 1.88 | 1.73 | 0.16 | 0.14 |
| Comp. compound (2-1) | 1.93 | 1.78 | 0.15 | 0.06 |

As shown above, the refractive index at a wavelength of 450 nm of the conventional material Alq₃ and the comparative compound (2-1) each are 1.88 and 1.93, while the diamine compounds having an azabenzooxazole ring structure represented by the general formula (a-1) of the present invention have a larger value of 2.35 to 2.58. Furthermore, the refractive index at a wavelength of 750 nm of the conventional material Alq₃ and the comparative compound (2-1) each are 1.73 and 1.78, while the diamine compound having an azabenzooxazole ring structure represented by the general formula (a-1) of the present invention each have a large value of 1.88 to 1.92 which is a value of 1.85 or higher. This indicates that an improvement in light extraction efficiency in an organic EL device can be expected by using a diamine compound having an azabenzooxazole ring structure represented by the general formula (a-1) preferably used in the organic EL device of the present invention as a constitutive material of a capping layer.

Further, the extinction coefficient at a wavelength of 400 nm to 410 nm of the conventional material Alq₃ and the comparative compound (2-1) each are 0.06 to 0.16, while the diamine compound having an azabenzooxazole ring structure represented by the general formula (a-1) of the present invention have a larger value of 0.80 to 1.09 which is a value of 0.2 or higher. This indicates that the capping layer formed with a diamine compound having an azabenzooxazole ring structure represented by the general formula (a-1) preferably used in the organic EL device of the present invention well absorbs light having a wavelength of 400 nm to 410 nm of sunlight, not to affect the materials inside the device.

### Example 9

Toluene solutions of the diamine compound having an azabenzooxazole ring structure represented by the general formula (a-1) were prepared at a concentration of 10⁻⁵ mol/L, and the absorbance thereof at a wavelength of 400 nm and 410 nm was measured with a UV-visible-near-infrared spectrophotometer (V-650, produced by JASCO Corporation). For the absorption coefficient, four different concentrations, 5 × 10⁻⁶ mol/L, 1 × 10⁻⁵ mol/L, 1.5 × 10⁻⁵ mol/L, and 2.0 × 10⁻⁵ mol/L of toluene solutions were prepared, and measured with a UV-visible-near-infrared spectrophotometer (V-650, produced by JASCO Corporation), and the absorption coefficient was calculated from the calibration curve. For comparison, the comparative compound (2-1) of the above structural formula and Alq₃ were also measured in the same manner. The measurement results are summarized in Table 2.

**[Table 2]**

| | Peak wavelength | Absorbance | Absorbance | Absorption coefficient |
|---|---|---|---|---|
| | (Amax) | (λ : 400 nm) | (λ : 410 nm) | |
| Compound (1-1) | 385 nm | 1.42 | 0.89 | 170036 |
| Compound (1-24) | 381 nm | 0.90 | 0.49 | 124444 |
| Compound (1-52) | 382 nm | 1.05 | 0.74 | 128480 |
| Compound (1-55) | 377 nm | 1.26 | 1.05 | 131818 |
| Compound (1-56) | 390 nm | 1.28 | 0.57 | 155816 |
| Compound (1-57) | 385 nm | 1.12 | 0.63 | 141216 |
| Alq₃ | 394 nm | 0.07 | 0.06 | 7518 |
| Comp. Compound (2-1) | 358 nm | 0.07 | 0.02 | 48856 |

As shown above, the absorbances at a wavelength of 400 nm and 410 nm of the conventional material Alq₃ and the comparative compound (2-1) each are 0.02 to 0.07, while the diamine compound having an azabenzooxazole ring structure represented by the general formula (a-1) of the present invention each have a larger value of 0.49 to 1.42 which is a value of 0.2 or higher. This indicates that a diamine compound having an azabenzooxazole ring structure represented by the general formula (a-1) of the present invention well absorbs light having a wavelength of 400 nm to 410 nm of sunlight.

As for the absorption coefficient, the diamine compounds having the azabenzooxazole ring structure represented by the general formula (a-1) of the present invention have a value of 100000 or higher, and have the absorption coefficient which is one digit larger than the absorption coefficient of the comparative compound. Specifically, this indicates that a diamine compound having an azabenzooxazole ring structure represented by the general formula (a-1) of the present invention well absorb the light under the same concentration condition, absorb the light better as the compounds become thicker in thin films, and are materials excellent in light resistance.

### Example 10

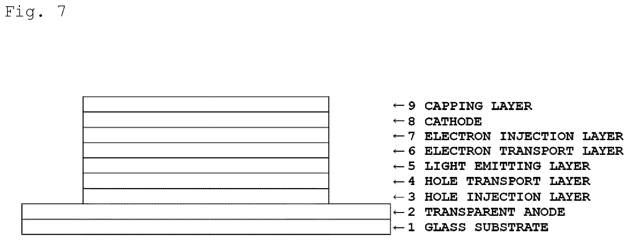

The organic EL device, as shown in Fig. 7, was fabricated by vapor-depositing a hole injection layer 3, a hole transport layer 4, a light emitting layer 5, an electron transport layer 6, an electron injection layer 7, a cathode 8, and a capping layer 9 in this order on a glass substrate 1 on which a reflective ITO electrode as a transparent anode 2 had been formed beforehand.

Specifically, as a metal anode 2, an ITO film with a thickness of 50 nm, a silver alloy reflective film with a thickness of 100 nm, and an ITO film with a thickness of 5 nm were deposited on the glass substrate 1 in this order. After ultrasonic cleaning in isopropyl alcohol for 20 minutes, the assembly was dried on a hot plate heated to 250°C for 10 minutes. After UV ozone treatment for 2 minutes, the glass substrate with ITO was installed in a vacuum vapor deposition apparatus, and the pressure was reduced to 0.001 Pa or lower. Subsequently, as the hole injection layer 3 covering the transparent anode 2, the electron acceptor (Acceptor-1) of the structural formula below and the compound (3-1) of the structural formula below were formed in a film thickness of 10 nm by dual vapor deposition at a vapor deposition rate ratio Acceptor-1 : compound (3-1) = 3:97. The hole transport layer 4 was formed on the hole injection layer 3 by forming the compounds (3-1) of the structural formula below in a film thickness of 140 nm. The light emitting layer 5 was formed on the hole transport layer 4 in a film thickness of 20 nm by dual vapor deposition of the compound (3-2) of the structural formula below and the compound (3-3) of the structural formula below at a vapor deposition rate ratio of compound (3-2):compound (3-3) = 5:95. The electron transport layer 6 was formed on the light emitting layer 5 in a film thickness of 30 nm by dual vapor deposition of the compound (3-4) of the structural formula below and the compound (3-5) of the structural formula below at a vapor deposition rate ratio of compound (3-4):compound (3-5) = 50:50. The electron injection layer 7 was formed on the electron transport layer 6 by forming lithium fluoride in a film thickness of 1 nm. On the electron injection layer 7, a magnesium silver alloy was formed as the cathode 8 in a film thickness of 12 nm. Finally, the compound (1-1) of Example 1 was formed as the capping layer 9 in a film thickness of 60 nm. The characteristics of the organic EL device thus fabricated were measured in the atmosphere at an ordinary temperature.

Table 3 summarizes the results of the emission characteristics measurements performed by applying a DC voltage to the fabricated organic EL device.

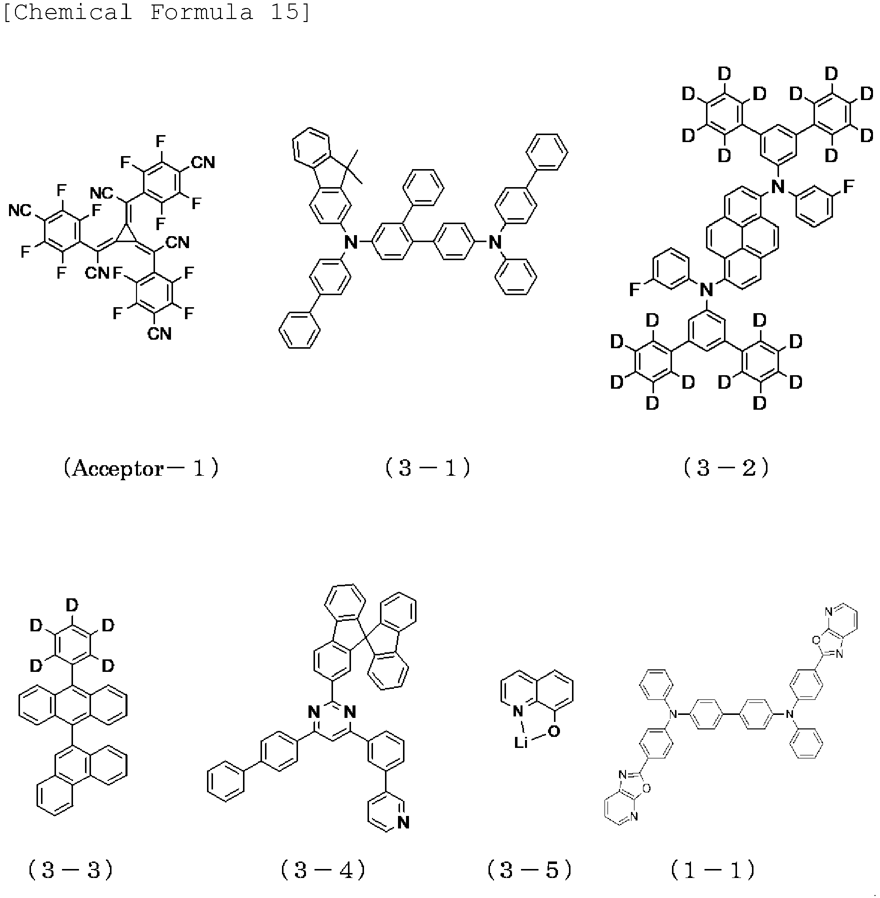

### Example 11

An organic EL device was fabricated under the same conditions as in Example 10 except that the compound (1-24) of Example 2 was used instead of the compound (1-1) of Example 1 as a material for the capping layer 9. The characteristics of the organic EL device thus fabricated were measured in the atmosphere at an ordinary temperature.

Table 3 summarizes the results of the emission characteristics measurements performed by applying a DC voltage to the fabricated organic EL device.

### Example 12

An organic EL device was fabricated under the same conditions as in Example 10 except that the compound (1-52) of Example 2 was used instead of the compound (1-1) of Example 1 as a material for the capping layer 9. The characteristics of the organic EL device thus fabricated were measured in the atmosphere at an ordinary temperature.

Table 3 summarizes the results of the emission characteristics measurements performed by applying a DC voltage to the fabricated organic EL device.

### Example 13

An organic EL device was fabricated under the same conditions as in Example 10 except that the compound (1-55) of Example 2 was used instead of the compound (1-1) of Example 1 as a material for the capping layer 9. The characteristics of the organic EL device thus fabricated were measured in the atmosphere at an ordinary temperature.

Table 3 summarizes the results of the emission characteristics measurements performed by applying a DC voltage to the fabricated organic EL device.

### Example 14

An organic EL device was fabricated under the same conditions as in Example 10 except that the compound (1-56) of Example 2 was used instead of the compound (1-1) of Example 1 as a material for the capping layer 9. The characteristics of the organic EL device thus fabricated were measured in the atmosphere at an ordinary temperature.

Table 3 summarizes the results of the emission characteristics measurements performed by applying a DC voltage to the fabricated organic EL device.

### Example 15

An organic EL device was fabricated under the same conditions as in Example 10 except that the compound (1-57) of Example 2 was used instead of the compound (1-1) of Example 1 as a material for the capping layer 9. The characteristics of the organic EL device thus fabricated were measured in the atmosphere at an ordinary temperature.

Table 3 summarizes the results of the emission characteristics measurements performed by applying a DC voltage to the fabricated organic EL device.

### Comparative Example 1

For comparison, an organic EL device was fabricated under the same conditions as in Example 10 except that Alq₃ was used instead of the compound (1-1) of Example 1 as a material for the capping layer 9. The characteristics of the organic EL device thus fabricated were measured in the atmosphere at an ordinary temperature.

Table 3 summarizes the results of the emission characteristics measurements performed by applying a DC voltage to the fabricated organic EL device.

### Comparative Example 2

For comparison, an organic EL device was fabricated under the same conditions as in Example 10 except that the comparative compound (2-1) of the above structural formula was used instead of the compound (1-1) of Example 1 as a material for the capping layer 9. The characteristics of the organic EL device thus fabricated were measured in the atmosphere at an ordinary temperature. Table 3 summarizes the results of the emission characteristics measurements performed by applying a DC voltage to the fabricated organic EL device.

Table 3 summarizes the results of the device lifetime measurements performed with the organic EL devices fabricated in Examples 10 to 15 and Comparative Examples 1 to 2. The device lifetime was measured as the time (attenuation to 95%) taken for the luminance to decay to 95%, with the initial luminance as 100%, when carrying out constant current drive of 10 mA/cm².

**[Table 3]**

| | Capping layer | Voltage [V] | Luminance [cd/m²] | Luminous efficiency [cd/A] | Power efficiency [1m/W] | Device lifetime |
|---|---|---|---|---|---|---|
| | | @10mA/cm² | @10mA/cm² | @10mA/cm² | @10mA/cm² | attenuation to 95% |
| Ex. 10 | Compound (1-1) | 3.63 | 826 | 8.27 | 7.16 | 149hr. |
| Ex. 11 | Compound (1-24) | 3.65 | 825 | 8.25 | 7.11 | 153hr. |
| Ex. 12 | Compound (1-52) | 3.64 | 833 | 8.33 | 7.20 | 162hr. |
| Ex. 13 | Compound (1-55) | 3.65 | 846 | 8.46 | 7.29 | 145hr. |
| Ex. 14 | Compound (1-56) | 3.65 | 826 | 8.27 | 7.13 | 150hr. |
| Ex. 15 | Compound (1-57) | 3.65 | 838 | 8.38 | 7.22 | 166hr. |
| Comp. Ex. 1 | Alq₃ | 3.65 | 714 | 7.14 | 6.15 | 114hr. |
| Com. Ex. 2 | Com. compound (2-1) | 3.66 | 735 | 7.34 | 6.30 | 133hr. |

As shown in Table 3, the driving voltage at a current density of 10 mA/cm² was almost the same for the devices in Comparative Examples 1 to 2 using the comparative compounds and Examples 10 to 15 using the compounds of the present invention. However, the luminance, the luminous efficiency, the power efficiency, and the device lifetime were significantly improved in the devices of Examples 10 to 15 using the compounds of the present invention, as compared to the devices of Comparative Examples 1 to 2 using the comparative compounds. This indicates that the light extraction efficiency of the organic EL devices can be significantly improved by including a diamine compound having an azabenzoazole ring structure represented by the general formula (a-1) of the present invention with a high refractive index in a capping layer.

### Industrial Applicability

As described above, a diamine compound having the azabenzoazole ring structure represented by the general formula (a-1) of the present invention has a high absorption coefficient, a high refractive index, which can significantly improve the light extraction efficiency, and the stability in thin film state, and therefore is excellent as a compound preferably used in a capping layer of an organic EL device. With the organic EL device fabricated by using the compound, a high efficiency can be obtained, and the durability and the light resistance can be improved so that sunlight is absorbed and does not affect the materials inside the device. The use of this compound, which has no absorption in the blue, green, and red wavelength regions, makes it particularly suitable for displaying clear, bright images with good color purity. For example, it is now possible to develop applications in home appliances and lighting.

### Reference Sign List

- 1: Glass substrate
- 2: Transparent anode
- 3: Hole injection layer
- 4: Hole transport layer
- 5: Light emitting layer
- 6: Electron transport layer
- 7: Electron injection layer
- 8: Cathode
- 9: Capping layer

## Claims

1. An diamine compound having an azabenzooxazole ring structure represented by the following general formula (a-1) : 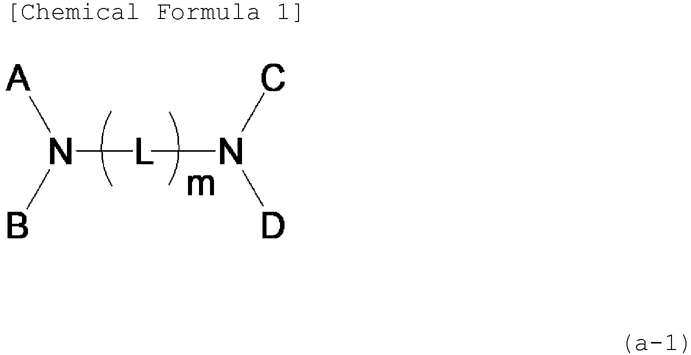
wherein, A, B, C and D may be the same or different, and represent a monovalent group having an azabenzooxazole group represented by the following structural formula (b-1), a substituted or unsubstituted aromatic hydrocarbon group, a substituted or unsubstituted aromatic heterocyclic group, or a substituted or unsubstituted condensed polycyclic aromatic group; at least one of A, B, C and D represents a monovalent group having an azabenzooxazole group represented by the following structural formula (b-1); L represents a divalent group of a substituted or unsubstituted aromatic hydrocarbon, a divalent group of a substituted or unsubstituted aromatic heterocyclic ring, or a divalent group of a substituted or unsubstituted condensed polycyclic aromatic, and may bind to each other with A, B, C or D via a single bond, a substituted or unsubstituted methylene, an oxygen atom, or a sulfur atom to form a ring; m represents an integer between 1 and 3, when m is an integer greater than or equal to 2, a plurality of L may be the same or different: 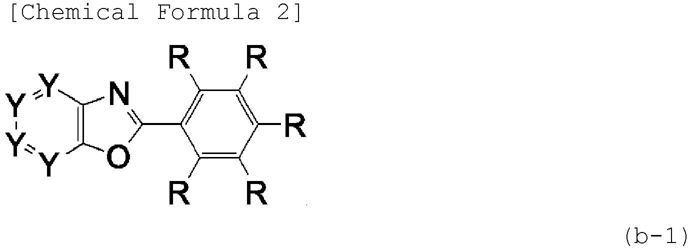
wherein Y may each be the same or different, and represents C-R or a nitrogen atom; at least one Y of a plurality of Y represents a nitrogen atom; R may each be the same or different, any one of a plurality of R is a linking group as a bonding site with the general formula (a-1), and represents a hydrogen atom, a deuterium atom, a chlorine atom, a cyano group, a nitro group, a trimethylsilyl group, a triphenylsilyl group, a linear or branched alkyl group of 1 to 6 carbon atoms that may have a substituent, a cycloalkyl group of 5 to 10 carbon atoms that may have a substituent, a linear or branched alkenyl group of 2 to 6 carbon atoms that may have a substituent, a linear or branched alkyloxy group of 1 to 6 carbon atoms that may have a substituent, a cycloalkyloxy group of 5 to 10 carbon atoms that may have a substituent, a substituted or unsubstituted aryloxy group, a substituted or unsubstituted aromatic hydrocarbon group, a substituted or unsubstituted aromatic heterocyclic group, or a substituted or unsubstituted condensed polycyclic aromatic group.

2. The diamine compound having an azabenzooxazole ring structure according to claim 1, wherein A, B, C and D in the general formula (a-1) are a monovalent group having an azabenzooxazole group represented by the structural formula (b-1), a substituted or unsubstituted phenyl group, a substituted or unsubstituted biphenyl group, a substituted or unsubstituted naphthyl group, a substituted or unsubstituted phenanthrenyl group, a substituted or unsubstituted carbazolyl group, a substituted or unsubstituted benzofuranyl group, a substituted or unsubstituted benzothienyl group, a substituted or unsubstituted indolyl group, a substituted or unsubstituted benzoimidazolyl group, a substituted or unsubstituted imidazopyridyl group, a substituted or unsubstituted benzoxazolyl group, a substituted or unsubstituted benzothiazolyl group, a substituted or unsubstituted dibenzofuranyl group, or a substituted or unsubstituted dibenzothienyl group.

3. The diamine compound having an azabenzooxazole ring structure according to claim 1 or 2, wherein the structural formula (b-1) is the following structural formula (b-2) or the following structural formula (b-3): 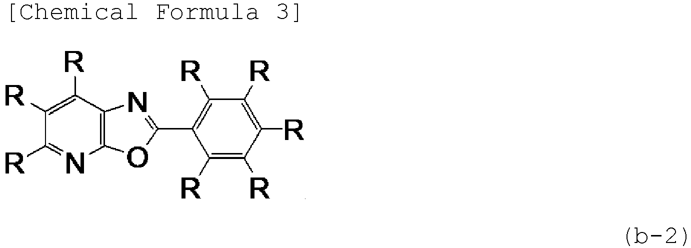
Wherein R is the same as defined by the structural formula (b-1): 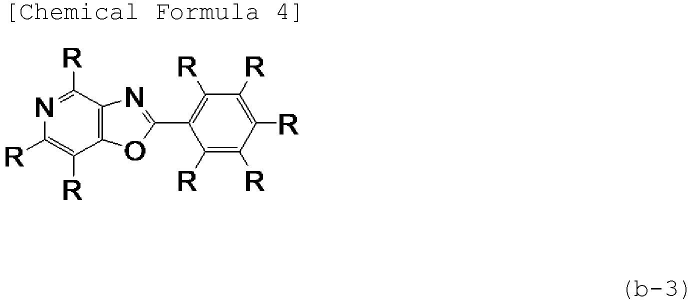
wherein R is the same as defined by the structural formula (b-1).

4. The diamine compound having an azabenzooxazole ring structure according to claim 1 or 2, wherein L in the general formula (a-1) is a substituted or unsubstituted phenylene group, a substituted or unsubstituted biphenylene group, or a substituted or unsubstituted naphthylene group.

5. The diamine compound having an azabenzooxazole ring structure according to claim 1 or 2, wherein m in the general formula (a-1) is 1 or 2.

6. The diamine compound having an azabenzooxazole ring structure according to claim 1 or 2, wherein the structural formula (b-1) is the following structural formula (b-4) or the following structural formula (b-5): 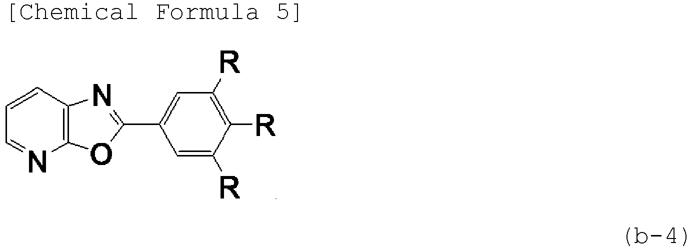
wherein, R is the same as defined by the structural formula (b-1): 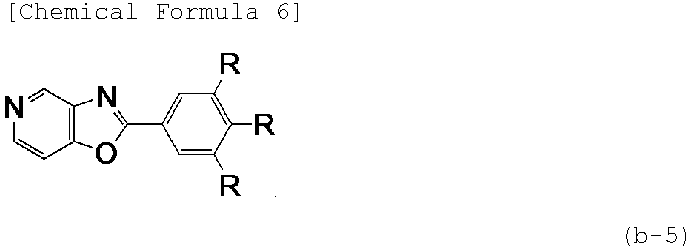
wherein, R is the same as defined by the structural formula (b-1).

7. The diamine compound having an azabenzooxazole ring structure according to claim 6, wherein only one of A, B, C and D in the general formula (a-1) is the structural formula (b-4) or the structural formula (b-5).

8. The diamine compound having an azabenzooxazole ring structure according to claim 6, wherein any two of A, B, C and D in the general formula (a-1) are the structural formula (b-4) or the structural formula (b-5).

9. The diamine compound having an azabenzooxazole ring structure according to claim 6, wherein A and B in the general formula (a-1) are the structural formula (b-4) or the structural formula (b-5).

10. The diamine compound having an azabenzooxazole ring structure according to claim 6, wherein A and C in the general formula (a-1) are the structural formula (b-4) or the structural formula (b-5).

11. An organic EL device comprising at least an anode electrode, a hole transport layer, a light emitting layer, an electron transport layer, a cathode electrode, and a capping layer in this order, wherein the capping layer includes the diamine compound having an azabenzooxazole ring structure according to any one of claims 1 to 10.

12. The organic EL device according to claim 11, wherein the capping layer has an extinction coefficient of 0.2 or higher at a wavelength in a range of 400 nm to 410 nm, and the diamine compound having an azabenzooxazole ring structure has an absorbance of 0.2 or higher in the absorption spectrum with a concentration of 10⁻⁵ mol/L at a wavelength in a range of 400 nm to 410 nm.

13. The organic EL device according to claim 11, wherein the capping layer has a refractive index of 1.85 or higher while light transmitted through the capping layer having a wavelength in a range of 450 to 750 nm.

14. The organic EL device according to claim 11, wherein in the case where the capping layer is a laminate or a mixed layer comprising two or more compounds, at least one of the compounds is the diamine compound having an azabenzooxazole ring structure.

15. An electronic apparatus or an electron device comprising a pair of electrodes and at least one organic layer sandwiched therebetween, wherein the organic layer includes the diamine compound having an azabenzooxazole ring structure according to any one of claims 1 to 10.
